# EUROPEAN PATENT SPECIFICATION

(11) **EP 3 289 352 B1**
(45) Date of publication and mention of the grant of the patent: **15.02.2023**
(21) Application number: 16725771.6
(22) Date of filing: 02.05.2016
(51) Int. Cl.: G01N 33/28, G01N 33/24, E21B 47/113, G01V 5/12, G01N 23/203

(54) **METHOD AND SYSTEM FOR IDENTIFYING FLUID TYPE INSIDE A CONDUIT**
VERFAHREN UND SYSTEM ZUR IDENTIFIZIERUNG DES FLUIDTYPS IN EINER LEITUNG
PROCÉDÉ ET SYSTÈME D'IDENTIFICATION DE TYPE DE FLUIDE À L'INTÉRIEUR D'UNE CONDUITE

(30) Priority: 30.04.2015 US 201562154955 P
(43) Date of publication of application: 07.03.2018
(60) Divisional application: 18179146.8
(73) Proprietor: Visuray Intech Ltd. (BVI), Road Town, Tortola (VG)
(72) Inventor: KAMBIZ, Safinya, Houston, TX 77056 (US); SPANNUTH, Melissa, 4008 Stavanger (NO); GUNN, Spencer, London E14 7DG (NO)
(74) Representative: Schneiders & Behrendt Bochum
(86) International application number: PCT/EP2016/059749
(87) International publication number: WO 2016/174260

(56) References cited:
- WO-A1-02/090954
- WO-A1-2005/022133
- US-A- 4 490 609
- US-A1- 2014 241 494

## Description

The present invention relates generally to a method and a system for identifying a fluid type inside a conduit.

The oil and gas industry has traditionally classified reservoirs by structures subdivided into geological units or pressure compartments in order to characterize fluids in a formation. Typically, fluid samples from varying depths of the well would either be retrieved from the well or analyzed *in situ.*

Retrieved fluid samples typically must be maintained under similar pressures and temperatures as the sampling environment in order to ensure that the properties of the sample do not change due to partial release of contained gas pressure. Previously known *in situ* methods have therefore relied upon optical or ultrasonic means for characterizing the fluid while it is still captured in various depths of the well.

Despite the development and advancement of various methods for determining formation fluid properties based on acquiring formation fluid samples from inside the wellbore, there remains a need to provide techniques capable of determining the composition of fluids without altering their properties or physical location by the action of sample collection or interrogation.

There are currently several *in situ* methods for fluid recognition available to operators, *viz.:*
1. Optical fluid characterization;
2. Ultrasonic methods, including time of flight, particulate count and flow rate (Doppler);
3. Electromagnetic methods consisting of irradiation of fluids by gamma rays from radioactive isotopes or x-rays from Bremsstrahlung and detection of transmitted radiation for analysis of the attenuation characteristics of said fluid; and
4. Electromagnetic methods consisting of irradiation of fluids by gamma rays from radioactive isotopes or x-rays from Bremsstrahlung and detection of scattered radiation for analysis of the attenuation characteristics of said fluid.

The optical fluid characterization approach involves passing key wavelengths of light (e.g., from infrared to ultraviolet) through the fluid to determine the attenuation coefficient of the fluid by analyzing the distribution of attenuation characteristics as a function of wavelength. By defining the characteristic distribution signature of the attenuation response to optical wavelengths of a fluid sample, the fluid under interrogation can be compared against a lookup table of known fluid attenuation characteristics and the most probable fluid type determined.

Ultrasonic methods involve transmission of pulses through the fluid between a transducer and a receiver over a predetermined distance, so that the time of flight can be measured and therefore the speed of sound within the fluid determined. Additionally, the ultrasonic properties of the fluid may be affected by the particulate content, thus the acoustic signature profile can be used to characterize the particulate content of the fluid. Other known means include using comparative time of flight paths in two directions through a moving fluid so that a Doppler shift can be measured and the speed of flow of the fluid determined.

Gamma and x-ray transmission methods consist of separating a sample of fluid from the main borehole and irradiating the sample with gamma or x-rays. A detector placed on the opposite side of the fluid compared to the radiation source then measures the amount and/or spectral energy distribution of the radiation that passes through the sample from said source. The radiation emitted by the source is attenuated in the fluid by an amount dependent upon the electron density profile of the fluid and the energy of the radiation. The resulting radiation transmitted through to the detector thus bears a signature of the composition of the intervening fluid. By comparing the amount and spectral energy distribution of the detected radiation against a database of known materials, the sample fluid can be identified.

Gamma and x-ray scattering methods consist of interrogating a fluid sample from the main borehole by irradiating the sample with gamma or x-rays, but without first isolating the sample from the borehole. A detector placed approximately adjacent to the radiation source then measures the amount and/or spectral energy distribution of the photons that scatter through the fluid from said source.

The radiation emitted by the source is scattered in the fluid by the amount related to the electron density profile of the fluid. Thus, the scattered radiation collected by the detector bears a signature of the composition of the borehole fluid. By comparing the amount and spectral energy distribution of the detected radiation against a database of known materials, the sample fluid can be identified.

The known prior art teaches a variety of techniques that use x-rays or other radiant energy to identify or obtain information about the fluid within the borehole of a water, oil or gas well, though none teach any method for identifying the fluid in front of a tool while said tool is moving through the well as described and claimed later in the application. Such a method provides the benefit that the fluid has not been altered prior to interrogation by mixing, movement of interfaces or otherwise disturbed by passage of the tool.

For example:
US 4 980 642 A describes a method for determining the dielectric constant of a fluid within a borehole surrounding a drill pipe. The method uses radar to determine the conductivity of the fluid surrounding the drill pipe within a borehole.
US 4 994 671 A teaches a methods and means for analyzing the composition of formation fluids through optical spectroscopic methods, primarily by way of comparison between the emitted wavelength of a source light and the detected wavelength after passing through a fluid sample.
US 5 276 656 A describes a method for using ultrasonic techniques for fluid identification and evaluation in boreholes. The method teaches a temporal evaluation of the ultrasonic properties of a fluid based on the speed of sound within the fluid, with an aim of determining the volume of said fluid by calculating the rate of change of said fluid properties as a function of volume.
US 4 628 725 A describes a method for using ultrasonic techniques for fluid identification and evaluation in a tubular conduit, specifically those surrounding a drill string. The method teaches of a means to determine ultrasonic properties of a fluid based on the speed of sound within the fluid.
US 4 947 683 A describes an apparatus which employs a Doppler borehole fluid measuring scheme. A rotating ultrasonic head is described that would be capable of measuring the interfaces between fluid types within a borehole. The concept of measurements based on the Doppler effect measurements being possible by the flow of gas bubbles within the fluid is also discussed.
US 7 675 029 A provides an apparatus that permits the measurement of x-ray backscattered photons from any solid surface inside of a borehole that refers to two-dimensional imaging techniques. US 7 675 029 A teaches use of spectroscopy and comparison with a materials database to determine the composition of the materials within the solid surface.
US 8 511 379 A describes a system and method for determining properties of a fluid based on the x-ray absorption of a fluid. The concept of transmission absorption with respect to a fluid is taught in addition to a multi-pixel array detector system which is employed to detect tracers within the fluid. However, it fails to teach of a system which combines all of the counts of each pixel such that the overall statistical noise can be reduced, thereby improving the quality of the signal.
US 7 807 962 A describes a system and method for determining properties of a fluid based on nuclear magnetic electromagnetic energy absorption of a fluid. The concept of transmission absorption with respect to a fluid is taught in addition to a system for guiding formation fluids from a pad into an assaying tubular section for sample analysis.
US 4 490 609 A discloses a method and apparatus for analyzing well fluids through irradiation by x-rays that aims to reduce the effects of the metal casing, cement and/or formation. Dual photon energy bands are used to independently measure the absorption from Thompson scattering and photoelectric effect. However, in the apparatus disclosed by US 4 490 609 A measurements are made in the central section of the apparatus, thus the fluid must be displaced around the tool itself before measurement. Moreover, US 4 490 609 A does not teach of a method which does not disturb the fluid prior to or at the time of measurement, as the fluid would already have been displaced around the tool housing itself.
US 2 261 539 A describes a method and apparatus for analyzing well fluids through irradiation of said fluids by gamma rays from an isotope. Similar to US 4 490 609 A, the detected counts result from attenuation of the source photons in the annular fluids between the tool and the borehole wall. However, US 2 261 539 A does not teach a method wherein the fluid if not disturbed prior to or at the time of measurement, since the fluid would already have been displaced around the tool housing itself.
US 7 075 062 A describes a system and method for determining properties of a fluid based on x-ray irradiation of a borehole fluid and attenuation measurements. The concept of the link between Compton scattering measurements and the electron density is discussed as well as the possibility of using multiple energy bands. US 7 075 062 A also suggests a system for guiding formation fluids from a pad into an assaying section of the apparatus for sample analysis, and the concept of removal of spurious data resulting from sand influx into the system is also considered.
US 7 507 952 A describes a system and method for determining properties of a fluid based on the x-ray absorption of the fluid. The concept of transmission absorption with respect to a fluid is taught in addition to the concept of a fluid comparator cell.
WO 02/090954 A1 relates to the use of X- or gamma- rays to determine the composition of materials. Moreover, the solution according to WO 02/090954 A1 can be used to measure bulk samples of material flowing through a pipeline.
WO 2005/022133 A1 discloses an apparatus for identifying material types in a target object in a fluid carrying pipe, comprising a light source arranged to emit high energy photons and a sensor unit arranged to register photons reflected from the target object.

There is, therefore, a longstanding need that remains unmet despite many prior unsuccessful attempts to achieve a forward-looking fluid analysis method that does not require transmission of a fluid sample from the wellbore into an apparatus, or otherwise disturbing the fluid.

In such a method, the radiation source and the imaging device would both need to be located within the tool housing near a low point of the apparatus, so that the fluid remains undisturbed and outside of the apparatus during measurement; in this manner, the various shortcomings of the prior art would be overcome. In addition, the prior art fails to teach of a mechanism through which an operator can anticipate fluid changes about to take place in front of the measuring tool prior to the tool reaching the interface; this approach would give an operator meaningful *a priori* knowledge regarding the status of the borehole, through the fluid composition of the borehole, in advance of the tool arriving at the sampled location.

The present inventors have now arrived at such a solution, example particulars of which are discussed below.

According to the invention, a method for identifying a fluid type inside a conduit according to claim 1 and a system for identifying a fluid type inside a conduit according to claim 2 are provided.

Preferred embodiments of the invention are described in the following with reference to the drawings. In the drawings:

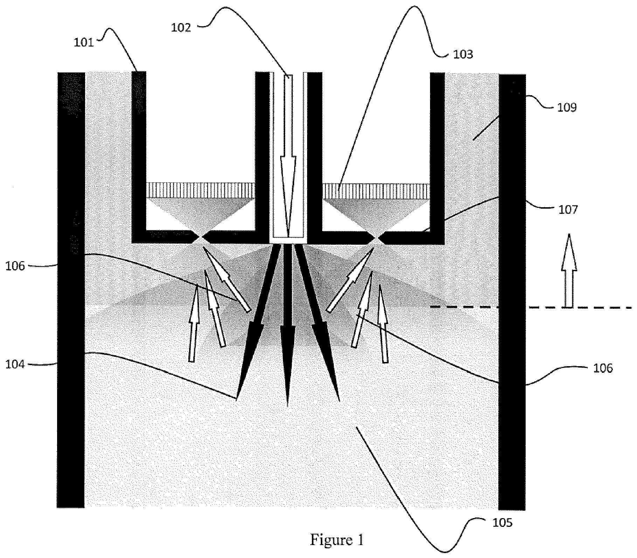

Figure 1 depicts an example embodiment comprising a tool disposed within a fluid-filled conduit containing an x-ray source, which is illuminating a volume of fluid separated by a fluid interface using x-rays; in this example, the scattered radiation resulting from the x-rays interaction with the fluid located in front of the tool is collected by a detector array or arrays;

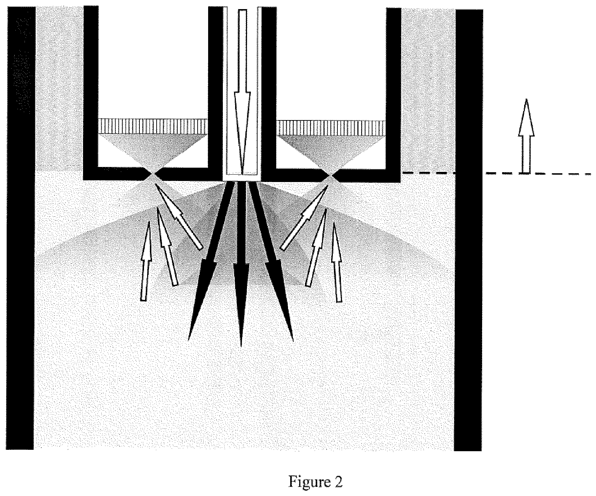

Figure 2 depicts the example embodiment of Figure 1, in which the tool has moved further into the conduit as illustrated by movement of the fluid interface into the fluid annulus between the tool and the conduit wall; consequently the scattering response of the fluid differs from the response of the interface between the two fluids in front of the tool;

There are no previously known technologies available on the market capable of providing an operator with non-disruptive means for determining the composition of a fluid or location of a fluid interface within a borehole with any significant level of detail with respect to the precise depth of a fluid interface or change.

In contrast, the methods and systems described and claimed herein allow an operator to determine the precise depth location and characteristic of a fluid in a conduit through a forward-looking fluid analysis method that does not require removal of the fluid sample from the wellbore into an isolation apparatus. In this manner, the fluid remains undisturbed and outside of the apparatus during measurement, particularly in a region in front of the apparatus as it is lowered into the conduit.

The objects of the invention are achieved by acquiring radiation backscattered from fluids disposed in front of the tool in the area of the conduit that has not been disturbed by the action of the measurement. The backscattered radiation is collected by one or more detector arrays and analyzed in detail using computational comparative characterization techniques.

With reference now to accompanying Figures 1 and 2, in an example embodiment primary x-rays 104 are produced by an x-ray tube 102 located within a pressure resistance tool housing 101. The primary x-rays illuminate a section of the well fluids 105, 109 in front of the tool 101, which results in the backscattering of radiation from both the Thompson and Compton effects. The scattered radiation 106 enters a collimation device 107 such as a pinhole, optical slot, array collimator or other collimation means, and falls upon a detector array 103 or arrays. The scattered radiation 106 is distributed across the surface of the detector array 103, which in further embodiments comprises a linear array or an area array.

As compared to a detector based upon a single scintillator crystal and photomultiplier tube, a pixel array effectively comprises many individual detectors, and as the nature of a collimator will always reduce the number of incoming counts as compared to configurations lacking collimation, ordinarily skilled artisans will readily appreciate that a pixel array achieves a number of key benefits.

For example, by distributing the total collected number of counts over a number of pixels, the statistical noise associated with each pixel can be reduced when the all of the counts associated with all of the detectors are combined as a single reading. An idealized detector would be capable of producing noise statistics identical to Poissonian distribution; however, by increasing the number of individual detectors measuring an acquisition, it is possible to reduce the overall signal to noise ratio within acceptable standards when considering the short acquisition times required to capture a reasonable data rate when considering that the tool is moving through the fluid and therefore through the conduit.

Once all of the individual counts associated with each pixel of each detector have been summed, it can be assumed that the statistical noise has been reduced to such an extent that the useable signal to noise ratio is sufficient to determine changes in the overall acquisitioned count rate such that any appreciable change (*e.g*., less than 1%) in fluid response would be detectable.

As the backscatter response of the fluid can be shown to be independent of the density of the fluid to the lowest order, it is possible to create a characteristic response of each of the fluid types an operator would expect to encounter in a fluid filled conduit.

According to the invention, the measured fluid response is then compared against a database of known fluid responses, and the specific fluid type determined as a function of the depth of the tool as it is moved through the conduit.

In a further embodiment still, the detector comprises a scintillator crystal coupled to a photo multiplier tube or photodiode. In yet another embodiment, the primary radiation 104 is produced by a chemical ionizing radiation source, such as a radioisotope.

In still further embodiments, the counts from each pixel of the detector array are not summed to obtain the total counts incident upon the entire detector, but instead individual pixels or groups of pixels are analyzed. This embodiment capitalizes on the highly localized region of space interrogated by each pixel in order to provide information about the spatial variations in fluid properties across the conduit. Furthermore, the scattered radiation recorded by different pixels or groups of pixels represents scattering through different angles as well as different attenuation path lengths. By comparing the signals received by different pixels with respect to these differences in scattering geometry, additional information can be obtained that may improve the fluid identification.

## Claims

1. A method for identifying a fluid type inside a conduit, said method comprising:
illuminating a fluid (105, 109) in a conduit using ionizing radiation (104) using a tool containing an x-ray source (102), which tool is disposed within the conduit;
determining a fluid response by detecting backscattered electromagnetic radiation (106) returned from the fluid (105, 109) located in front of the tool using one or more electromagnetic radiation sensors (103), wherein the x-ray source (102) and the one or more electromagnetic radiation sensors (103) are located within a housing (101) of the tool,
identifying the fluid type as a function of the depth of the tool in the conduit by comparing the fluid response against a database of known fluid responses as the tool is moved through the conduit,
**characterized in that** the x-ray source (102) and the one or more electromagnetic radiation sensors (103) are located at the lower end of the tool.

2. A system for identifying a fluid type inside a conduit, said system comprising:
a tool containing an x-ray source (102) for illuminating a fluid (105, 109) in a conduit using ionizing radiation (104);
means for determining a fluid response using one or more electromagnetic radiation sensors (103) for detecting backscattered electromagnetic radiation (106) returned from the fluid (105, 109) located in front of the tool, wherein the x-ray source (102) and the one or more electromagnetic radiation sensors (103) are located within a housing (101) of the tool; and
means for identifying the fluid type as a function of the depth of the tool in the conduit by comparing the fluid response against a database of known fluid responses as the tool is moved through the conduit,
**characterized in that** the x-ray source (102) and the one or more electromagnetic radiation sensors (103) are located at the lower end of the tool.

## Patentansprüche

1. Verfahren zum Identifizieren eines Fluidtyps innerhalb einer Leitung, das Verfahren umfassend:
Beleuchten eines Fluids (105, 109) in einer Leitung mithilfe von ionisierender Strahlung (104) unter Verwendung eines Werkzeugs, das eine Röntgenstrahlquelle (102) enthält, welches Werkzeug in der Leitung angeordnet ist;
Bestimmen einer Fluidreaktion durch Detektieren von rückgestreuter elektromagnetischer Strahlung (106) vor dem Werkzeug, die von dem Fluid (105, 109) zurückgeworfen wird, unter Verwendung von einem oder mehr Sensoren für elektromagnetische Strahlung (103), wobei die Röntgenstrahlquelle (102) und der eine oder mehr Sensoren für elektromagnetische Strahlung (103) sich innerhalb eines Gehäuses (101) des Werkzeugs befinden,
Identifizieren des Fluidtyps abhängig von der Tiefe des Werkzeugs in der Leitung durch Abgleichen der Fluidreaktion mit einer Datenbank bekannter Fluidreaktionen, wenn das Werkzeug durch die Leitung bewegt wird,
**dadurch gekennzeichnet, dass** die Röntgenstrahlquelle (102) und der eine oder mehr Sensor(en) für elektromagnetische Strahlung (103) sich am unteren Ende des Werkzeugs befinden.

2. System zum Identifizieren eines Fluidtyps innerhalb einer Leitung, das System aufweisend:
ein Werkzeug, das eine Röntgenstrahlquelle (102) enthält, um unter Verwendung ionisierender Strahlung (104) ein Fluid (105, 109) in einer Leitung zu beleuchten;
Einrichtungen zum Bestimmen einer Fluidreaktion unter Verwendung von einem oder mehr Sensoren (103) für elektromagnetische Strahlung zum Detektieren von rückgestreuter elektromagnetischer Strahlung (106), die von dem Fluid (105, 109) zurückgeworden wurde, vor dem Werkzeug, wobei die Röntgenstrahlquelle (102) und der eine oder mehr Sensor(en) für elektromagnetische Strahlung (103) sich innerhalb eines Gehäuses (101) des Werkzeugs befinden; und
Einrichtungen zum Identifizieren des Fluidtyps abhängig von der Tiefe des Werkzeugs in der Leitung durch Abgleichen der Fluidreaktion mit einer Datenbank bekannter Fluidreaktionen, wenn das Werkzeug durch die Leitung bewegt wird,
**dadurch gekennzeichnet, dass** die Röntgenstrahlquelle (102) und der eine oder mehr elektromagnetische Sensor(en) (103) sich am unteren Ende des Werkzeugs befinden.

## Revendications

1. Procédé pour identifier un type de fluide à l'intérieur d'un conduit, ledit procédé comprenant :
l'illumination d'un fluide (105, 109) dans un conduit en utilisant un rayonnement ionisant (104) en utilisant un outil contenant une source de rayons X (102), lequel outil est disposé à l'intérieur du conduit ;
la détermination d'une réponse du fluide en détectant le rayonnement électromagnétique rétrodiffusé (106) renvoyé par le fluide (105, 109) situé devant l'outil en utilisant un ou plusieurs capteurs de rayonnement électromagnétique (103), la source de rayons X (102) et les un ou plusieurs capteurs de rayonnement électromagnétique (103) étant situés à l'intérieur d'un boîtier (101) de l'outil,
l'identification du type de fluide en fonction de la profondeur de l'outil dans le conduit en comparant la réponse du fluide à une base de données de réponses de fluide connues lorsque l'outil est déplacé dans le conduit,
**caractérisé en ce que** la source de rayons X (102) et les un ou plusieurs capteurs de rayonnement électromagnétique (103) sont situés à l'extrémité inférieure de l'outil.

2. Système pour identifier un type de fluide à l'intérieur d'un conduit, ledit système comprenant :
un outil contenant une source de rayons X (102) pour illuminer un fluide (105, 109) dans un conduit en utilisant un rayonnement ionisant (104) ;
des moyens pour déterminer une réponse du fluide en utilisant un ou plusieurs capteurs de rayonnement électromagnétique (103) pour détecter le rayonnement électromagnétique rétrodiffusé (106) renvoyé par le fluide (105, 109) situé devant l'outil, la source de rayons X (102) et les un ou plusieurs capteurs de rayonnement électromagnétique (103) étant situés à l'intérieur d'un boîtier (101) de l'outil ; et
des moyens pour identifier le type de fluide en fonction de la profondeur de l'outil dans le conduit en comparant la réponse du fluide à une base de données de réponses de fluide connues lorsque l'outil est déplacé dans le conduit,
**caractérisé en ce que** la source de rayons X (102) et les un ou plusieurs capteurs de rayonnement électromagnétique (103) sont situés à l'extrémité inférieure de l'outil.
